(19) Europäisches Patentamt
European Patent Office
Office européen des brevets

(11) Veröffentlichungsnummer: **0 200 066**
**A1**

(12) # EUROPÄISCHE PATENTANMELDUNG

(21) Anmeldenummer: **86105086.2**

(22) Anmeldetag: **14.04.86**

(51) Int. Cl.$^4$: **C 07 D 251/46**

(30) Priorität: **27.04.85 DE 3515286**

(43) Veröffentlichungstag der Anmeldung:
**05.11.86 Patentblatt 86/45**

(84) Benannte Vertragsstaaten:
**AT BE CH DE FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Riebel, Hans-Jochem, Dr.**
**In der Beek 92**
**D-5600 Wuppertal 1(DE)**

(54) **Verfahren zur Herstellung von 2-Cyanamino-4,6-dialkoxy-1,3,5-triazinen.**

(57) Die Erfindung betrifft ein neues Verfahren zur Herstellung von 2-Cyanamino-4,6-dialkoxy-1,3,5-triazinen der allgemeinen Formel (I)

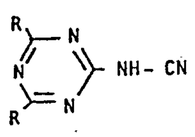

(I)

in welcher
R für Alkoxy steht,
das dadurch gekennzeichnet ist, daß man 2-Cyanamino-4,6-dichlor-1,3,5-triazine der Formel (II)

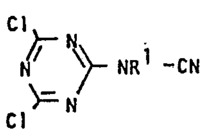

(II)

in welcher
$R^1$ für ein Äquivalent eines Alkalimetallions steht,
mit mindestens der zweifachen molaren Menge Alkoholat der Formel (III)

RMe (III)

in welcher
R die oben angegebene Bedeutung hat und
Me für ein Äquivalent eines Alkalimetallions steht,
in Gegenwart von Verdünnungsmitteln umsetzt, anschließend mit Wasser versetzt und ansäuert.

EP 0 200 066 A1

BAYER AKTIENGESELLSCHAFT 5090 Leverkusen, Bayerwerk

Konzernverwaltung RP Bi/mü
Patentabteilung IVa/ZP

## Verfahren zur Herstellung von 2-Cyanamino-4,6-dialkoxy-1,3,5-triazinen

Die vorliegende Erfindung betrifft ein neues Verfahren zur Herstellung von 2-Cyanamino-4,6-dialkoxy-1,3,5-triazinen, welche als Zwischenprodukte für die Herstellung von Herbiziden und Pflanzenwachstumsregulatoren verwendet werden können.

Es ist bereits bekannt, daß 2-Cyanamino-1,3,5-triazine durch Umsetzung von Alkalimetall- oder Erdalkalimetall-Salzen von Cyanamid mit den entsprechenden 2-Halogen-1,3,5-triazinen erhalten werden (vgl. z.B. DE-OS 3 334 455 / EP-A-121 082). Dieses Verfahren ist jedoch wegen unbefriedigender Herstellungsmethoden für die benötigten Ausgangsprodukte nur sehr begrenzt anwendbar. Es besteht daher ein Bedarf an einem neuen, breit anwendbaren Herstellungsverfahren für 2-Cyanamino-4,6-dialkoxy-1,3,5-triazine.

Es wurde nun gefunden, daß man 2-Cyanamino-4,6-dialkoxy-1,3,5-triazine der allgemeinen Formel (I)

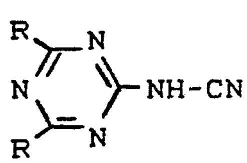

(I)

in welcher

R für Alkoxy steht,

erhält, wenn man 2-Cyanamino-4,6-dichlor-1,3,5-triazine der Formel (II)

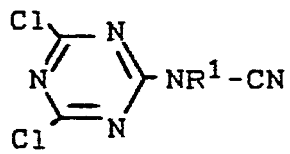

(II)

in welcher

$R^1$ für ein Äquivalent eines Alkalimetallions steht,

mit mindestens der zweifachen molaren Menge Alkoholat der Formel (III)

RMe (III)

in welcher

R die oben angegebene Bedeutung hat und

Me für ein Äquivalent eines Alkalimetallions steht,

in Gegenwart von Verdünnungsmitteln umsetzt, anschließend mit Wasser versetzt und ansäuert.

Überraschenderweise gelingt es mit diesem erfindungsgemäßen Verfahren, die Verbindungen der Formel (I) in guten Ausbeuten zu erhalten. Bei der Herstellung der Verbindungen der Formel (I) nach dem Stand der Technik aus Alkalimetall- bzw. Erdalkalimetall-Salzen von Cyanamid und den entsprechenden Halogen-1,3,5-triazinen sind die Ausbeuten sehr unbefriedigend.

Bevorzugt werden mit Hilfe des erfindungsgemäßen Verfahrens die Verbindungen der Formel (I) hergestellt, in welcher

R für Alkoxy mit 1 bis 6 Kohlenstoffatomen steht.

Besonders bevorzugt werden diejenigen Verbindungen der Formel (I) hergestellt, in welcher

R für Methoxy, Ethoxy, n-Propoxy, i-Propoxy, n-Butoxy, i-Butoxy, sec.-Butoxy oder tert.-Butoxy steht.

Ganz besonders bevorzugt werden diejenigen Verbindungen der Formel (I) hergestellt, in welcher

R für Methoxy, Ethoxy, n-Propoxy, i-Propoxy oder n-Butoxy steht.

Verwendet man beispielsweise für das erfindungsgemäße Verfahren das Natriumsalz von 2-Cyanamino-4,6-dichlor-1,3,5-triazin und Natriumethylat als Ausgangsstoffe, so kann die Reaktion durch das folgende Formelschema wiedergegeben werden:

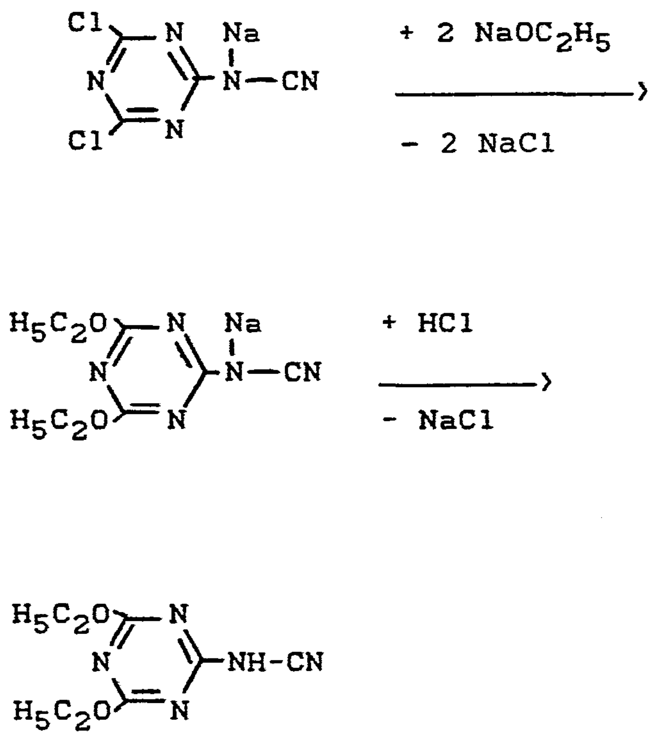


Die als Ausgangsstoffe für das erfinderische Verfahren zu verwendenden 2-Cyanamino-4,6-dichlor-1,3,5-triazine sind durch die Formel (II) allgemein definiert. In dieser Formel steht R bevorzugt für diejenigen Reste, welche oben im Rahmen der Substituentendefinition der Formel (I) vorzugsweise bzw. als insbesondere bevorzugt an-

gegeben sind. $R^1$ steht in dieser Formel für ein Äquivalent eines Alkalimetallions, wie insbesondere für ein Natrium- oder Kaliumion. Bevorzugt steht $R^1$ für ein Natriumion.

Als Beispiele für die Verbindungen der Formel (II) seien genannt:
das Natrium- oder Kaliumsalz von 2-Cyanamino-4,6-dichlor-1,3,5-triazin.

Die Verbindungen der Formel (II) sind bekannt (vgl z.B. DE-AS 2 050 948).

Die weiterhin als Ausgangsstoffe für das erfindungsgemäße Verfahren zu verwendenden Alkoholate sind durch die Formel (III ) allgemein definiert. In dieser Formel hat R vorzugsweise bzw. insbesondere die gleichen Bedeutungen, wie sie oben im Rahmen der Substituentendefinition der Formel (I) vorzugsweise bzw. als insbesondere bevorzugt genannt sind. Me steht in dieser Formel vorzugsweise für ein Natrium- oder Kaliumion.

Als Beispiele für die Verbindungen der Formel (III) seien beispielsweise genannt:
Natrium-methylat, -ethylat, -n-propylat, -i-propylat, -n-butylat, -i-butylat, -sec.-butylat und -tert.-butylat und die entsprechenden Kalium-Derivate.

Die Verbindungen der Formel (III) sind allgemein bekannte Verbindungen der organischen Chemie.

Das erfindungsgemäße Verfahren wird in Gegenwart von Verdünnungsmitteln durchgeführt.
Hierzu gehören insbesondere aliphatische und aromatische Kohlenwasserstoffe, wie Pentan, Hexan, Heptan, Cyclohexan, Petrolether, Benzin, Ligroin, Benzol, Toluol, Xylol; Alkohole, wie Methanol, Ethanol, n-Propanol, i-Propanol, n-Butanol, i-Butanol, sec.-Butanol und tert.-Butanol; Ketone, wie Aceton, Methylethyl-, Methylisopropyl- und Methylisobutylketon; Nitrile, wie z.B. Acetonitril und Propionitril; Amide, wie z.B. Dimethylformamid, Dimethylacetamid und N-Methylpyrrolidon, sowie Dimethylsulfoxid, Tetramethylensulfon und Hexamethylphosphorsäuretriamid. Bevorzugt werden die entsprechenden Alkohole der eingesetzten Alkoholate der Formel (III) verwendet.

Das erfindungsgemäße Verfahren wird im allgemeinen bei Temperaturen zwischen 0°C und 60°C, vorzugsweise zwischen 0°C und 40°C durchgeführt. Die Umsetzungen werden im allgemeinen bei Normaldruck durchgeführt.

Bei der Durchführung des erfindungsgemäßen Verfahrens setzt man auf ein Mol der Verbindung der Formel (II) 2,0 bis 2,5 Mol, vorzugsweise 2,0 bis 2,3 Mol Alkoholat der Formel (III) ein. Die Aufarbeitung der Verbindung der Formel (I) geschieht nach üblichen Methoden. Nach beendeter Zugabe der Ausgangsstoffe wird noch kurze Zeit oder auch mehrere Stunden bei 15°C bis 25°C nachgerührt. Anschließend wird eingeengt und der Rückstand mit Wasser

versetzt und mit einer Mineralsäure, wie z.B. Salzsäure angesäuert. Die Verbindungen der Formel (I) fallen im allgemeinen in kristalliner Form an.

Die nach den erfindungsgemäßen Verfahren herzustellenden 2-Cyanamino-4,6-dialkoxy-1,3,5-triazine können als Zwischenprodukte für die Herstellung von Guanidin-Derivaten, die als Herbizide und Pflanzenwuchsregulatoren wirksam sind, eingesetzt werden (vgl. EP-OS 121 082).

## Herstellungsbeispiele

### Beispiel 1

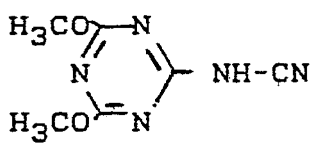

Zu einer Suspension von 30 g (0,14 Mol) 2-Cyanamino-4,6-dichlor-1,3,5-triazin-Natriumsalz in 200 ml Methanol tropft man eine Lösung von 6,9 g (0,3 Mol) Natrium in 50 ml Methanol in der Weise, daß eine Temperatur von 40°C nicht überschritten wird. Anschließend wird bei 20°C 2 Stunden nachgerührt, abfiltriert und das Filtrat eingeengt. Der Rückstand wird in 50 ml Wasser gelöst und mit Salzsäure angesäuert. Die entstandenen Kristalle werden abgesaugt und getrocknet.

Man erhält 22,6 g (89 % der Theorie) 2-Cyanamino-4,6-dimethoxy-1,3,5-triazin. Das Produkt wird durch $^1H$-NMR-Spektren charakterisiert.

## Patentansprüche

1). Verfahren zur Herstellung von 2-Cyanamino-4,6-dialkoxy-1,3,5-triazinen der allgemeinen Formel (I)

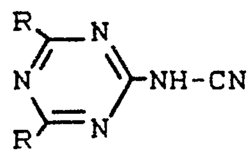 (I)

in welcher

R für Alkoxy steht,

dadurch gekennzeichnet, daß man 2-Cyanamino-4,6-dichlor-1,3,5-triazine der Formel (II)

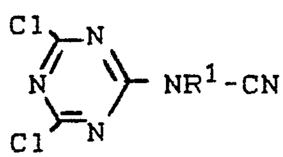 (II)

in welcher

$R^1$ für ein Äquivalent eins Alkalimetallions steht,

mit mindestens der zweifachen molaren Menge Alkoholat der Formel (III)

RMe (III)

in welcher

R die oben angegebene Bedeutung und

Me für ein Äquivalent eines Alkalimetallions steht,

in Gegenwart von Verdünnungsmitteln umsetzt, anschließend mit Wasser versetzt und ansäuert.

Europäisches Patentamt

**EUROPÄISCHER RECHERCHENBERICHT**

0200066

Nummer der Anmeldung

EP 86105086.2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | GB - A - 1 052 567 (LONZA)<br>* Anspruch 1; Beispiele 1,2 *<br>-- | 1 | C 07 D 251/46 |
| A | CHEMICAL ABSTRACTS, Band 94, Nr. 19, 11. Mai 1981, Columbus, Ohio, USA<br>DOVLATYAN, V.V.; KHACHATRYAN, L.A.; AMBARTSUMYAN, E.N. "Action of acid chlorides, hydrochloric acid, and carbon dioxide on cyanamino-s-triazine salts"<br>Seite 656, Spalte 2, Zusammenfassung-Nr. 156 874q<br>& Arm. Khim. Zh. 1980, 33(9), 755-8<br>---- | 1 | |
| | | | **RECHERCHIERTE SACHGEBIETE (Int. Cl.4)** |
| | | | C 07 D 251/00 |

Der vorliegende Recherchenbericht wurde für alle Patentansprüche erstellt.

| Recherchenort | Abschlußdatum der Recherche | Prüfer |
|---|---|---|
| WIEN | 16-07-1986 | HAMMER |

KATEGORIE DER GENANNTEN DOKUMENTEN

- X : von besonderer Bedeutung allein betrachtet
- Y : von besonderer Bedeutung in Verbindung mit einer anderen Veröffentlichung derselben Kategorie
- A : technologischer Hintergrund
- O : nichtschriftliche Offenbarung
- P : Zwischenliteratur
- T : der Erfindung zugrunde liegende Theorien oder Grundsätze

- E : älteres Patentdokument, das jedoch erst am oder nach dem Anmeldedatum veröffentlicht worden ist
- D : in der Anmeldung angeführtes Dokument
- L : aus andern Gründen angeführtes Dokument

- & : Mitglied der gleichen Patentfamilie, übereinstimmendes Dokument

EPA Form 1503 03 82